# EUROPÄISCHE PATENTANMELDUNG

(11) **EP 2 425 842 A1**
(43) Veröffentlichungstag der Anmeldung: **07.03.2012**
(21) Anmeldenummer: 11178280.1
(22) Anmeldetag: 22.08.2011
(51) Int. Cl.: A61K 33/18, A61K 36/15, A61K 36/534, A61K 36/61, A61K 47/44, A61K 9/00

(54) **Zusammensetzung zur symptomatischen Lokaltherapie der Nasenschleimhaut und Nasentropfen enthaltend diese Zusammensetzung**

(30) Priorität: 02.09.2010 DE 202010012096 U
(71) Anmelder: Lorentz, Eckart, 53225 Bonn (DE)
(72) Erfinder: Lorentz, Eckart, Dr. med., 53225 Bonn (DE)
(74) Vertreter: Wagner, Matthias

(57) **Zusammenfassung**

Die Erfindung betrifft eine Zusammensetzung zur symptomatischen Lokaltherapie der Nasenschleimhaut sowie Nasentropfen enthaltend eine derartige Zusammensetzung.

## Beschreibung

Die Erfindung betrifft eine Zusammensetzung zur symptomatischen Lokaltherapie der Nasenschleimhaut.

Die Nase ist auf mannigfache Weise an der Respiration beteiligt. So findet in der Nase die Präparation der Atemluft statt, um sie für die Überführung der Gase in den Lungenalveolen geeignet zu machen. Voraussetzung der Gaswechsel ist eine Wasserdampfsättigung der eingeatmeten Luft bei Alveolentemperatur, wobei die Nase an der Präparierung, insbesondere der Anfeuchtung und Erwärmung der Atemluft entscheidend beteiligt ist. Ferner soll die Atemluft frei von Beimengungen unerwünschter Stoffe sein. Die Funktionen der Nase bestehen also in Anfeuchtung, Erwärmung und Reinigung der Atemluft. Bei der eingeatmeten Atemluft herrscht im Nasenrachen eine Temperatur von 31 - 34° C bei etwa 98 - 100 % Wasserdampfsättigung.

Diese Funktionen der Nase hängen entscheidend von der ständigen Sekretbedeckung der Nasenschleimhaut ab. Dieses Sekret wird in Schleimdrüsen der Nasenschleimhaut gebildet und ist für die Anfeuchtung der Atemluft verantwortlich.

Für die Reinigungsfunktion der Nase und den Weitertransport des Nasensekretes ist das so genannte Flimmerepithel verantwortlich, welches sich auf der Nasenschleimhaut befindet und sich ständig von vorn nach hinten bewegt. Der Schleim der Nase bindet Fremdkörper, Schadstoffe und Erreger und wird vom Flimmerepithel in Richtung Rachen bewegt. Der Schleim wird anschließend verschluckt und die Magensäure inaktiviert Schadstoffe und Erreger, was als mukoziliäre Clearance bezeichnet wird. Auf diese Weise reinigt die Nase bis zu 20.000 Liter Atemluft täglich von Schmutzpartikeln, aber auch von Viren, Bakterien, Hefen und Pilzen.

Der Nasenschleim wird von so genannten Becherzellen der Nasenschleimhaut gebildet und enthält bakterienhemmende Substanzen und Antikörper gegen Viren. Die mukoziliäre Clearance müssen also Erreger überwinden, bevor eine Entzündungs- bzw. Immunantwort des Organismus ausgelöst wird.

Aus dem Vorgenannten ergibt sich die extreme Wichtigkeit einer funktionsfähigen Nasenschleimhaut. Trockene oder geschädigte Nasenschleimhaut kann hingegen die Reinigung und Anfeuchtung der Atemluft nicht mehr vollständig erfüllen. Fehlender oder zu zäher Schleim macht die Zilien bewegungsunfähig, so dass ein Abtransport von Krankheitserregern unmöglich wird. Erkältungs-und Grippeviren können in der Folge eindringen und sich ausbreiten.

Die ungenügende Sekretfunktion der Nase, die bis zum völligen Versiegen des Sekretstromes führen kann, ist ein sehr unangenehmes Krankheitsbild. Es befällt bevorzugt Menschen jenseits des 50. Lebensjahres, deren Nase durch atrophische und/oder sklerotische und/oder narbige Prozesse verändert ist. Beim Betrachten des Naseninneren fallen Bezirke mit fest haftenden Borken und Krusten auf, wobei manchmal sogar Borkenbildung im Nasenrachen bis hinunter zum Kehlkopf beobachtet wird, wobei der Mundrachen nur durch eine ausgesprochene Trockenheit der Schleimhaut auffällt.

Durch die verminderte Resistenz der Schleimhäute der Atemwege ergibt sich eine Störung der Atemwegsfunktion, die häufig zu chronischer Reizung oder Entzündung von Rachen, Kehlkopf oder Bronchien führen kann, jedoch ursächlich von der Funktionseinschränkung der Nasenschleimhaut ausgeht.

Als Therapiemaßnahmen werden neben eventuell notwendigen operativen Eingriffen auch symptomatische Lokaltherapien der Nasenschleimhaut erwogen, um die Nasensekretion und Ziliarfunktion wieder in Gang zu bringen.

Aufgabe der Erfindung ist es daher, eine Zusammensetzung zur symptomatischen Lokaltherapie der Nasenschleimhaut vorzuschlagen, die bei möglichst einfacher Anwendung die Nasensekretion und Ziliarfunktion stimulieren und wieder herzustellen vermag.

Zu Lösung der gestellten Aufgabe wird erfindungsgemäß eine Zusammensetzung mit den Merkmalen des Patentanspruchs 1 vorgeschlagen.

Vorteilhafte Ausgestaltungen und Weiterbildungen der Erfindung sind Gegenstand der abhängigen Ansprüche.

Die im Rahmen der Erfindung vorgeschlagene Zusammensetzung enthält 1 Teil Jod in 300 - 500 Teilen Neutralöl, vorzugsweise in Form einer Emulsion.

Jod führt zu einer vermehrten serösen Schleimproduktion, dem so genannten Jodschnupfen, was bei einer trockenen Nasenschleimhautentzündung mit verdicktem, zähem Schleim und damit gestörter mukoziliärer Clearance zwecks Verdünnung des Schleimes außerordentlich erwünscht ist. Außerdem wirkt Jod durch seine Reizwirkung auf die Schleimhaut durchblutungsfördernd und erzielt einen ebenfalls erwünschten desinfizierenden Effekt bei verminderter oder gestörter Clearance.

Die Verabreichung einer erfindungsgemäßen Zusammensetzung mit einem Teil Jod in 300 - 500 Teilen Neutralöl ist für die Erzielung der vorgenannten mit dem Jod assoziierten Wirkungen ausreichend, wobei die Gesamtmenge an verabreichtem Jod gleichwohl so niedrig liegt, dass sie von der überwiegenden Zahl von Patienten problemlos toleriert wird.

Da die erfindungsgemäße Zusammensetzung als mengenmäßig wesentlichsten Bestandteil 300 - 500 Teile Neutralöl aufweist, wird neben der längeren Verweildauer der erfindungsgemäßen Zusammensetzung auf der Nasenschleimhaut im Vergleich zu wässrigen Lösungen der weitere Effekt erreicht, dass sich das Neutralöl wie ein schützender Film über die geschädigte Schleimhaut legt und sich die unterhalb des Ölfilmes liegende geschädigte Schleimhaut überraschend schnell und effektiv zu regenerieren vermag. Der Ölfilm auf der zu regenerierenden Schleimhaut bewirkt auch eine länger andauernde Feuchtigkeit derselben.

Schließlich wirkt sich die aufgrund der Verwendung von Neutralöl gegebene längere Einwirkungszeit auch günstig auf das Aufweichen eventuell vorhandener Borken und Krusten in der Nase aus, die ansonsten leicht zu Nasenbluten führen können.

Selbstverständlich können anstellen von Neutralöl auch andere pflanzliche und tierische Öle sowie ölige Lösungen verwendet werden, wobei jedoch Neutralöl, d.h. mittelkettige Triglyzeride (C8 - C12) bevorzugt wird.

Neutralöl haftet gut auf einer trockenen und geschädigten Nasenschleimhaut, überzieht diese mit einem schützenden Film, stößt damit die Regeneration an und ermöglicht die Wiederaufnahme der Bewegung des Flimmerepithels, so dass eine mukoziliäre Clearance wieder in Gang kommt.

Neutralöl reizt ferner die Schleimhäute nicht, behindert auch nicht die Ziliarbewegung und benötigt insbesondere keinen Zusatz von Konservierungsstoffen und ist auch in den geringen Mengen, die von der Schleimhaut aufgenommen wird, vom Organismus im Rahmen des normalen Stoffwechsels leicht abbaubar.

Gegenüber anderen zur symptomatischen Lokaltherapie der Nasenschleimhaut verwendeten Darreichungsformen, beispielsweise Salben, erzielt die erfindungsgemäße Zusammensetzung, insbesondere wenn sie in Form von Nasentropfen verwendet wird, wesentlich bessere Wirkungen. Salben lassen nämlich nur eine Applikation im Nasenvorhof zu, da ihr Viskositätsgrad eine Ausbreitung auf der Schleimhaut verhindert. Ferner wirkt sich der Einfluss von Salben ungünstig auf die Ziliarbewegung aus.

Zur Einstellung eines für den Patienten angenehmen Geruchs und Geschmacks sowie zur Erzeugung des Gefühls einer freieren Nasenatmung kann die erfindungsgemäße Zusammensetzung ferner ein ätherisches Öl umfassen, beispielsweise Pfefferminzöl, Latschenkieferöl, Eukalyptusöl oder Abmischungen derselben.

Erfindungsgemäß wird vorgeschlagen, dass etwa 40 - 80 Teile ätherisches Öl pro eingesetztem Teil Jod verwendet sind.

Bevorzugt wird die erfindungsgemäße Zusammensetzung in Form von Nasentropfen oder Nasenspray verabreicht.

Ausführungsbeispiel:
Eine Zusammensetzung zur symptomatischen Lokaltherapie der Nasenschleimhaut mit besonders guter Wirkung zur Behandlung der Rhino-Pharyngo-Laryngitis sicca mit Reizhusten weist folgende Rezeptur auf:
   Jod 0,05
   Ol Menthae pip gtt 3,00
   Ol neutrale ad 20,00

Überraschenderweise hat sich gezeigt, dass durch Behandlung der oberen Atemwege, insbesondere der Nasenschleimhaut mit einer derartigen Zusammensetzung in Form von Nasentropfen auch Krankheitsbilder wie Reizhusten, Heiserkeit, Rachenentzündungen therapiert werden konnten, da als jeweiliger Auslöser dieser Beschwerden eine ausgetrocknete oder gestörte Nasenschleimhaut ausgemacht werden konnte, die sich nach Behandlung mit der erfindungsgemäßen Zusammensetzung so weit regenerierte, dass die Beschwerden aufgehoben wurden.

## Patentansprüche

1. Zusammensetzung zur symptomatischen Lokaltherapie der Nasenschleimhaut, enthaltend 1 Teil Jod in 300 - 500 Teilen Neutralöl.

2. Zusammensetzung nach Anspruch 1, **dadurch gekennzeichnet, dass** sie ferner ein ätherisches Öl enthält.

3. Zusammensetzung nach Anspruch 2, **dadurch gekennzeichnet, dass** als ätherisches Öl Pfefferminzöl, Latschenkieferöl, Eukalyptusöl oder eine Abmischung derselben eingesetzt ist.

4. Zusammensetzung nach Anspruch 2 oder 3, **dadurch gekennzeichnet, dass** 40 - 80 Teile ätherisches Öl pro eingesetztem Teil Jod verwendet sind.

5. Zusammensetzung nach einem der vorhergehenden Ansprüche, enthaltend:
0,05 Teile Jod;
3,00 Teile Pfefferminzöl; und
20,00 Teile Neutralöl.

6. Nasentropfen zur symptomatischen Lokaltherapie der Nasenschleimhaut, **gekennzeichnet durch** eine Zusammensetzung gemäß einem der vorangehenden Ansprüche.
